Europäisches Patentamt
European Patent Office
Office européen des brevets

(19)

(11) Publication number: 0 091 302 A1

(12) # EUROPEAN PATENT APPLICATION

(21) Application number: 83301876.5

(22) Date of filing: 31.03.83

(51) Int. Cl.³: C 07 D 499/00
C 07 D 499/62, A 61 K 31/43
//C07C79/38, C07C79/24,
C07C59/00

(30) Priority: 03.04.82 GB 8209982

(43) Date of publication of application: 12.10.83 Bulletin 83/41

(84) Designated Contracting States: BE CH DE FR GB IT LI NL SE

(71) Applicant: BEECHAM GROUP PLC
Beecham House Great West Road
Brentford Middlesex(GB)

(72) Inventor: Burton, George
14 Windborough Road
Carshalton Surrey(GB)

(72) Inventor: Lashford, Andrew Gerard
15 Lake Road North Roath Park
Cardiff Wales(GB)

(74) Representative: Hesketh, Alan, Dr. et al,
European Patent Attorney Beecham Pharmaceuticals
Great Burgh Yew Tree Bottom Road
Epsom, Surrey, KT18 5XQ(GB)

(54) **Beta-lactam derivatives.**

(57) A compound of formula (I) or a pharmaceutically acceptable salt or *in vivo* hydrolysable ester thereof:

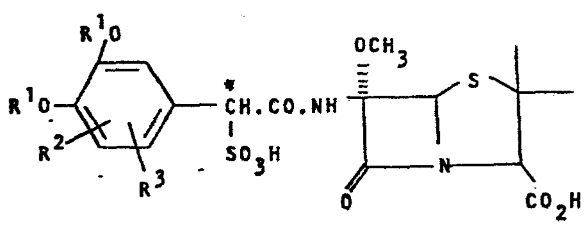

(I)

wherein $R^1$ is hydrogen or $C_{1-6}$ alkylcarbonyl or $C_{1-6}$ alkoxycarbonyl; $R^2$ is hydrogen, halogen, hydroxy, $C_{1-6}$ alkoxy, nitro, amino, $C_{1-6}$ alkyl, $C_{1-6}$ haloalkyl, $C_{1-6}$ alkylcarbonyloxy, $C_{1-6}$ alkyl sulphonylamino, carboxy, cyano, or $C_{1-6}$ alkoxycarbonyl; $R^3$ is halogen, hydroxy, $C_{1-6}$ alkoxy, nitro, amino, $C_{1-6}$ alkyl, $C_{1-6}$ haloalkyl, $C_{1-6}$ alkycarbonyloxy, or $C_{1-6}$ alkyl sulphonylamino, carboxy, cyano, or $C_{1-6}$ alkyloxycarbonyl; a process for the preparation of such compounds and pharmaceutical compositions comprising them.

EP 0 091 302 A1

# β LACTAM DERIVATIVES

This invention relates to a class of β-lactam derivatives which have antibacterial activity and are of value in the treatment of infections in animals, including mammals and in particular humans, caused by a wide range of organisms, particularly Gram-negative organisms. In particular the invention relates to a small class of 6α-methoxy penicillin derivatives. The invention also relates to a process for the preparation of such compounds, and to pharmaceutical compositions comprising them.

According to the present invention there is provided a compound of formula (I) or a pharmaceutically acceptable salt or *in vivo* hydrolysable ester thereof:

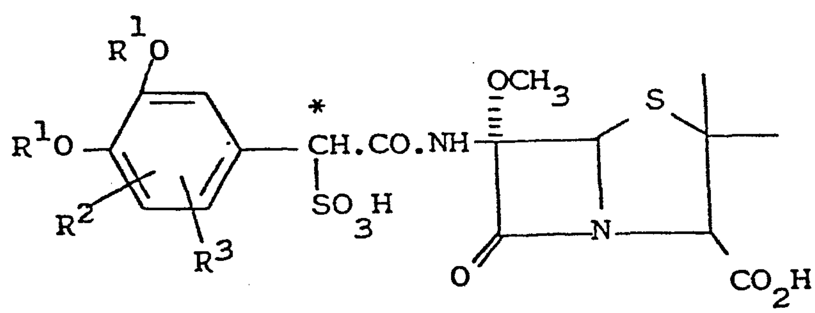

(I)

wherein $R^1$ is hydrogen or $C_{1-6}$ alkylcarbonyl or $C_{1-6}$ alkoxycarbonyl; $R^2$ is hydrogen, halogen, hydroxy, $C_{1-6}$ alkoxy, nitro, amino, $C_{1-6}$ alkyl, $C_{1-6}$ haloalkyl, $C_{1-6}$ alkylcarbonyloxy, $C_{1-6}$ alkyl sulphonylamino,

carboxy, cyano, or $C_{1-6}$ alkoxycarbonyl; $R^3$ is halogen, hydroxy, $C_{1-6}$ alkoxy, nitro, amino, $C_{1-6}$ alkyl, $C_{1-6}$ haloalkyl, $C_{1-6}$ alkylcarbonyloxy, or $C_{1-6}$ alkyl sulphonylamino, carboxy, cyano, or $C_{1-6}$ alkyloxycarbonyl.

The carbon atom marked * in formula (I) is asymmetric and the compound may be derived from the side-chain having a R, S, or R, S configuration at that position. All forms of compound (I) are included in this invention. Suitably, the carbon atom marked * is in the R-configuration.

Suitably the substituent $R^2$ is hydrogen, halogen, hydroxy, $C_{1-6}$ alkoxy, nitro, $C_{1-6}$ alkylcarbonyloxy, carboxy or $C_{1-6}$ alkoxycarbonyl.

Suitably the substituent $R^3$ is halogen, hydroxy, $C_{1-6}$ alkoxy, nitro, amino, $C_{1-6}$ alkylcarbonyloxy, carboxy or $C_{1-6}$ alkyl.

Preferably $R^1$ is hydrogen or acetyl.

Preferably $R^2$ is hydrogen.

Most preferably $R^2$ is hydrogen and is in the 2-position relative to the carbon atom marked * in formula (I).

Preferably $R^3$ is chlorine, bromine, hydroxy, amino, nitro, cyano, carboxy, methyl, ethyl or acetoxy.

Examples of suitable pharmaceutically acceptable <u>in vivo</u> hydrolysable ester groups include those which break down readily in the human body to leave the parent acid or its salts, for example acyloxyalkyl groups, such as acetoxymethyl, pivaloyloxymethyl,

α-acetoxyethyl and α-pivaloyloxyethyl groups; alkoxycarbonyloxyalkyl groups, such as ethoxycarbonyloxymethyl and α-ethoxycarbonyloxyethyl; dialkylaminoalkyl groups, such as dimethylaminomethyl, dimethylaminoethyl, diethylaminomethyl or diethylaminoethyl; and lactone groups such as phthalidyl or dimethoxyphthalidyl.

Suitable pharmaceutically acceptable salts of the compounds of formula (I) include metal salts, eg aluminium, alkali metal salts such as sodium or potassium, alkaline earth metal salts such as calcium or magnesium and ammonium or substituted ammonium salts, for example those with lower alkylamines such as triethylamine, hydroxy-lower alkylamines such as 2-hydroxyethylamine, bis-(2-hydroxyethyl)-amine or tri-(2-hydroxyethyl)-amine, cycloalkylamines such as bicyclohexylamine, or with procaine, dibenzylpiperidine, N-benzyl-β-phenethylamine, dehydroabrietylamine, N,N'-bisdehydroabietylamine, ethylenediamine, or bases of the pyridine type such as pyridine, collidine or quinoline.

One preferred sub-group within the present invention provides a compound of formula (II) or a pharmaceutically acceptable salt or <u>in vivo</u> hydrolysable ester thereof:

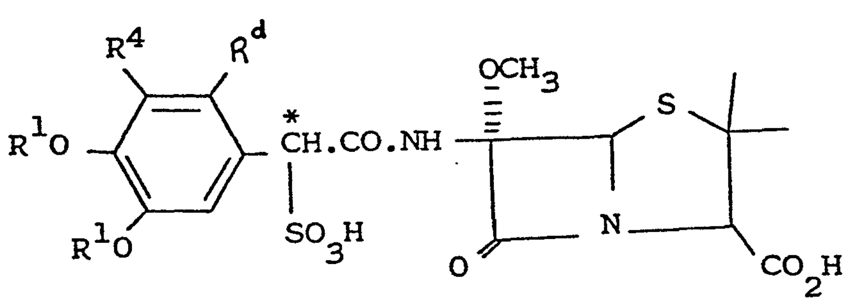

(II)

wherein $R^1$ is as defined with respect to formula (I); $R^4$ and $R^d$ independently represent hydrogen, amino nitro, halogen, hydroxy, $C_{1-6}$ alkoxy, $C_{1-6}$ alkoxycarbonyl, $C_{1-6}$ alkyl cyano, carboxy or $C_{1-6}$ alkylcarbonyloxy.

Suitably at least one $R^4$ or $R^d$ is chloro, amino, nitro, hydroxy, methyl, methoxy, acetoxy or carboxy.

Preferably one of $R^4$ and $R^d$ is hydrogen. When used herein the term 'lower' refers to groups containing from 1 to 6 carbon atoms.

Specific compounds within this invention include:

6,β-[2-(3,4-dihydroxy-5-nitrophenyl)-2-sulphoacetamido]-6,α-methoxypenicillanic acid;

6,β-[2-(5-amino-3,4-dihydroxyphenyl)-2-sulphoacetamido]-6,α-methoxypenicillanic acid;

6,β-[2-(3-chloro-4,5-dihydroxyphenyl)-2-sulphoacetamido]-6,α-methoxypenicillanic acid;

6,β-[2-(2-chloro-4,5-diacetoxyphenyl)-2-sulphoacetamido-6,α-methoxypenicillanic acid;

6,β-[2-(4,5-diacetoxy-2-methylphenyl)-2-sulphoacetamido]-6,α-methoxypenicillanic acid;

6,β-[2-(4,5-diacetoxy-3-methylphenyl)-2-sulphoacetamido-6,α-methoxypenicillanic acid; and

6,β-[2-(4,5-diacetoxy-2,3-dichlorophenyl)-2-sulphoacetamido-6,α-methoxypenicillanic acid.

The compounds of formula (I) may be prepared by reacting a compound of formula (III):

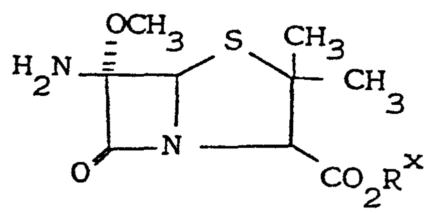

(III)

wherein the amino group is optionally substituted with a group which permits acylation to take place, and wherein $R^x$ is hydrogen or a carboxyl-blocking group, with an N-acylating derivative of an acid of formula (IV):

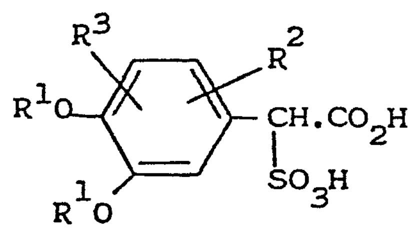

(IV)

wherein $R^1$, $R^2$ and $R^3$ are defined with respect to formula (I) above and wherein any reactive groups may be protected; and thereafter, if necessary, carrying out one or more of the following steps:

i) removing any carboxyl-blocking group $R^x$;
ii) removing any substituent on the amide group;
iii) removing any protectiong group on the acid of formula (IV);
iv) converting the product into a salt or <u>in vivo</u> hydrolysable ester thereof.

Suitable groups which permit acylation to take place and which are optionally present on the amino group of the starting material of the formula (II) include N-silyl, N-stannyl and N-phosphorus groups, for example trialkylsilyl groups such as trimethylsilyl, trialkyltin groups such as tri-*n*-butyltin, groups of

formula $-P.R^aR^b$ wherein $R^a$ is an alkyl, haloalkyl, aryl, aralkyl, alkoxy, haloalkyl, aryl, aralkyl, alkoxy, haloalkoxy, aryloxy, aralkyloxy or dialkylamino group, $R^b$ is the same as $R^a$ or is halogen or $R^a$ and $R^b$ together form a ring; suitable such phosphorus groups being $-P(OC_2H_5)_2$, $-P(C_2H_5)_2$,

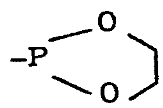

and

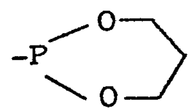

Suitable carboxyl-blocking derivatives for the group $-CO_2R^x$ in formula (III) include salts and ester derivatives of the carboxylic acid. The derivative is preferably one which may readily be cleaved at a later stage of the reaction. Suitable salts include metal salts, such as those with sodium, potassium and lithium, and tertiary amine salts, such as those with trilower-alkylamines, N-ethylpiperidine, 2,6-lutidine, pyridine, N-methylpyrrolidine, dimethylpiperazine. A preferred salt is with triethylamine.

Suitable ester-forming carboxyl-blocking groups are those which may be removed under conventional conditions. Such groups for $R^x$ include benzyl, p-methoxybenzyl, 2,4,6-trimethylbenzyl, 3,5-di-t-butyl-4-hydroxy-benzyl, benzoylmethyl, p-nitrobenzyl, 4-pyridylmethyl, 2,2,2-trichloroethyl, 2,2,2-tribromoethyl, t-butyl, t-amyl, diphenylmethyl, triphenylmethyl, adamantyl, 2-benzyloxyphenyl, 4-methylthiophenyl, tetrahydrofur-2-yl, tetrahydropyran-2-yl, pentachlorophenyl, p-toluenesulphonylethyl, methoxymethyl, a silyl, stannyl or phosphorus-containing group, such as described above, an oxime radical of formula $-N=CHR^o$ where $R^o$ is aryl or heterocyclic, or an in vivo hydrolysable ester radical such as defined above.

The carboxyl group may be regenerated from any of the above esters by usual methods appropriate to the particular $R^x$ group, for example, acid - and base - catalysed hydrolysis, or by enzymically - catalysed hydrolysis, or by hydrogenation.

A reactive N-acylating derivative of the acid (IV) is employed in the above process. The choice of reactive derivative will of course be influenced by the chemical nature of the substituents of the acid.

Suitable N-acylating derivatives include an acid halide, preferably the acid chloride or bromide. Acylation with an acid halide may be affected in the presence of an acid binding agent for example, tertiary amine (such as triethylamine or dimethylaniline), an inorganic base (such as calcium carbonate or sodium bicarbonate) or an oxirane, which binds hydrogen halide liberated in the acylation reaction. The oxirane is preferably a $(C_{1-6})$-1,2,alkylene oxide - such as ethylene oxide or propylene oxide. The acylation reaction using an acid halide may be carried out at a temperature in th range -50°C to +50°C, preferably -20°C to +20°C, in aqueous or non-aqueous media such as aqueous acetone, aqueous tetrahydroform, ethyl, acetate, dimethylacetamide, dimethylformamide, acetonitrile, dichloromethane, 1,2-dichloroethane, or mixtures thereof. Alternatively, the reaction may be carried out in an unstable emulsion of water-immiscible solvent, especially an aliphatic ester or ketone, such as methyl isobutyl ketone or butyl acetate.

The acid halide may be prepared by reacting the acid (IV) or a salt thereof with a halogenating (eg. chlorinating or brominating) agent such as phosphorus pentachloride, thionyl chloride or oxalyl chloride.

The acid halide may also be prepared by reacting an appropriately substituted phenylacetyl halide, wherein any reactive substituents are protected, with a sulphonating agent, such as, for example, the sulphur trioxide-dioxane complex.

Alternatively, the N-acylating derivative of the acid (IV) may be a symmetrical or mixed anhydride. Suitable mixed anhydrides are alkoxyformic anhydrides, or anhydrides with, for example carbonic acid monoesters, trimethyl acetic acid, thioacetic acid, diphenylacetic acid, benzoic acid, phosphorus acids (such as phosphoric or phosphorous acids), sulphuric acid or aliphatic or aromatic sulphonic acids (such as p-toluenesulphonic acid). The mixed or symmetrical anhydrides may be generated using N-ethoxycarbonyl-2-ethoxy-1,2-dihydroquinoline. When a symmetrical anhydride is employed, the reaction may be carried out in the presence of 2,4-lutidine as catalyst.

Alternative N-acylating derivatives of acid (IV) are the acid azide, or activated esters such as esters with 2-mercaptopyridine, cyanomethanol, p-nitrophenol, 2,4-dinitrophenol, thiophenol, halophenols, including pentachlorophenol, monomethoxyphenol or 8-hydroxyquinoline; or amides such as N-acylsaccharins or N-acylphthalimides; or an alkylidene iminoester prepared by reaction of the acid (IV) with an oxime.

Other reactive N-acylating derivatives of the acid (IV) include the reactive intermediate formed by reaction in situ with a condensing agent such as a carbodiimide, for example N,N′-diethyl-, dipropyl- or diisopropylcarbodiimide, N,N'-di-cyclohexylcarbodiimide or N-ethyl-N'- -dimethylaminopropylcarbodiimide; a suitable carbonyl compound, for example N,N'-carbonyldiimidazole or N,N'-carbonylditriazole; an

isoxazolinium salt, for example N-ethyl-5-phenylisoxazolinium-2-sulphonate or N-*t*-butyl-5-methylisoxazolinium perchlorate; or an N-alkoxycarbonyl-2-alkoxy-1,2-dihydroquinoline, such as N-ethoxycarbonyl-2-ethoxy-1,2-dihydroquinoline. Other condensing agents include Lewis acids (for example $BBr_3$ - $C_6H_6$); or a phosphoric acid condensing agent such as diethylphosphorylcyanide. The condensation reaction is preferably carried out in an organic reaction medium, for example methylene chloride, dimethylformamide, acetonitrile, alcohol, benzene, dioxan, or tetrahydrofuran.

The starting material of formula (III) is disclosed in British Patent No. 1,339,007.

Compounds of formula (I) may also be prepared by reacting a compound of formula (V):

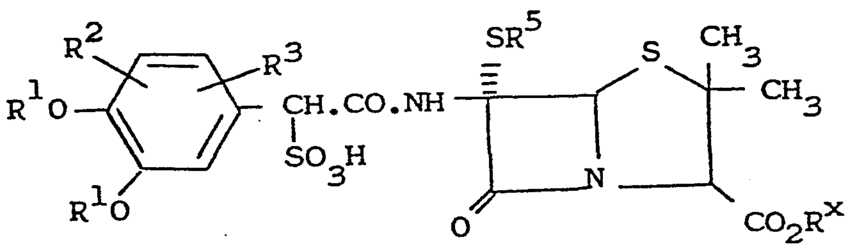

(V)

wherein $R^1$, $R^2$, $R^3$ and $R^x$ are as defined above and $R^5$ is $C_{1-6}$ alkyl, aryl or benzyl; with methanol in the presence of a metal ion, such as mercury, lead, silver, cadmium, thallium, tellurium or bismuth.

This reaction may generally be carried out at a temperature of from -50°C to +25°C, but is conveniently carried out between -5°C and +25°C. Any suitable solvent may be employed. It is generally convenient however to use methanol as the solvent. Preferably $R^5$ in formula (V) represents methyl. Any reactive groups in the side-chain will suitably be protected prior to the present process and deprotected subsequently.

Compounds of formula (I) may also be prepared by:

a) treating a compound of formula (VI):

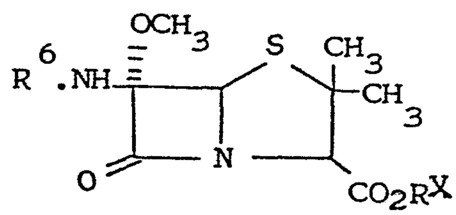

(VI)

wherein $R^x$ is a carboxyl-blocking group, and $R^6$ is an acyl group, in particular an acyl group derived from the side-chain of a natural penicillin, such as benzyl penicillin or phenoxymethyl penicillin; with an agent forming an imino halide;

b) treating the imino halide with a compound to introduce a group $QR_f$ on the imino carbon atom, wherein Q is oxygen, sulphur or nitrogen and $R_f$ is an alkyl group of from 5 to 14 carbon atoms, to form an iminoether, iminothioether, or amidine (when Q is O, S, or N respectively);

c) reacting with an N-acylating derivative of an acid of formula (IV) and wherein any reactive groups may be protected;

d) treating with water; and

e) optionally removing the carboxyl-blocking group $R^x$ and any protecting groups on the side-chain.

A suitable agent for preparing an imino halide is an acid halide in the presence of an acid binding agent such as a tertiary amine, eg. pyridine, triethylamine, or N,N-dimethylaniline. Examples of suitable acid halides are phosphorus pentachloride, phosgene, phosphorous pentabromide, phosphorus oxychloride, oxalyl chloride and p-toluene sulphonic acid chloride.

Phosphorus pentachloride and phosphorus oxychloride are preferred. The reaction may be conducted under cooling, preferably at temperatures from 0°C to -30°C when phosphorous pentachloride is employed. The amount of the tertiary amine is preferably 3 - 5 mols per mol of phosphorus pentachloride. It is also preferable to use the phosphorus halide in an amount slightly in excess of that of the starting material.

The resulting imino compounds are then treated to introduce a $-QR_f$ group onto the imino carbon atom. This is preferably effected by reacting the imino halide with a corresponding alcohol. Examples of suitable alcohols for reaction wih the imino halide are aliphatic alcohols containing from 1 to 12 carbon atoms, preferably 1 to 5 carbon atoms, such as methanol, ethanol, propanol, isopropyl alcohol, amyl alcohol and butyl alcohol, and aralkyl alcohols such as benzyl alcohol and 2-phenylethanol.

The reaction of the alcohol with the imino halide is preferably effected in the presence of an acid binding agent, such as a tertiary amine, preferably pyridine, and the reaction is usually carried out without isolating the imino halide from the reaction mixture.

Finally, the product is treated with water. The water treatment may be conducted together with the isolation of the desired material. That is the reaction mixture may be added to water or a saturated aqueous solution of sodium chloride and then the aqueous layer formed is separated from the organic solvent layer.

The antibiotic compounds according to the invention may be formulated for administration in any convenient way for use in human or veterinary medicine, by analogy with other antibiotics, and the invention therefore includes within its scope a pharmaceutical composition comprising a compound of formula (I) above together with a pharmaceutical carrier or excipient.

The composition may be formulated for administration by any route, such as oral topical or parenteral. The compositions may be in the form of tablets, capsules, powders, granules, lozenges, creams or liquid preparations, such as oral or sterile parenteral solutions or suspensions.

Tablets and capsules for administration may be in unit dose presentation form, and may contain conventional excipients such as binding agents, for example syrup, acacia, gelatin, sorbitol, tragacanth, or polyvinylpyrollidone: fillers, for example lactose, sugar, maize-starch, calcium phosphate, sorbitol or glycine, tabletting lubricants, for example magnesium stearate, talc, polyethylene glycol or silica; disintegrants, for example potato starch; or acceptable wetting agents such as sodium lauryl sulphate. The tablets may be coated according to methods well known in normal pharmaceutical practice. Oral liquid preparations may be in the form of, for example, aqueous or oily suspensions, solutions, emulsions,

syrups or elixirs, or may be presented as a dry product for reconstitution with water or other suitable vehicle before use. Such liquid preparations may contain conventional additives, such as suspending agents, for example sorbitol, methyl cellulose, glucose syrup, gelatin, hydroxyethyl cellulose, carboxymethyl cellulose, aluminium stearate gel or hydrogenated edible fats, emulsifying agents, for example lecithin, sorbitan monooleate, or acacia; non-aqueous vehicles (which may include edible oils), for example almond oil, oily esters such as glycerine, propylene glycol, or ethyl alcohol; preservatives, for example methyl or propyl p-hydroxybenzoate or sorbic acid, and, if desired, conventional flavouring or colouring agents.

Suppositories will contain conventional suppository bases, e.g. cocoa-butter or other glyceride.

For parenteral administration, fluid unit dosage forms are prepared utilizing the compound and a sterile vehicle, water being preferred. The compound, depending on the vehicle and concentration used, can be either suspended or dissolved in the vehicle. In preparing solutions the compound can be dissolved in water for injection and filter sterilised before filling into a suitable vial or ampoule and sealing. Advantageously, agents such as a local anaesthetic, preservative and buffering agents can be dissolved in the vehicle. To enhance the stability, the composition can be frozen after filling into the vial and the water removed under vacuum. The dry lyophilized powder is then sealed in the vial and an accompanying vial of water for injection may be supplied to reconstitute the liquid prior to use. Parental suspensions are prepared in substantially the same manner except that the compound is suspended in the vehicle instead of being

dissolved and sterilization cannot be accomplished by filtration. The compound can be sterilised by exposure to ethylene oxide before suspending in the sterile vehicle. Advantageously, a surfactant or wetting agent is included in the composition to facilitate uniform distribution of the compound.

The compositions may contain from 0.1% by weight, preferably from 10-60% by weight, of the active material, depending on the method of administration. Where the composition comprise dosage units, each unit will preferably contain from 50-500 mg of the active ingredient. The dosage as employed for adult human treatment will preferably range from 100 to 300 mg per day, for instance 1500 mg per day depending on the route and frequency of administration.

The compound of formula (I) may be the sole therapeutic agent in the compositions of the invention or a combination with other antibiotics or with a β-lactamase inhibitor may be employed.

Advantageously, the compositions also comprise a compound of formula (VII) or a pharmaceutically acceptable salt or ester thereof:

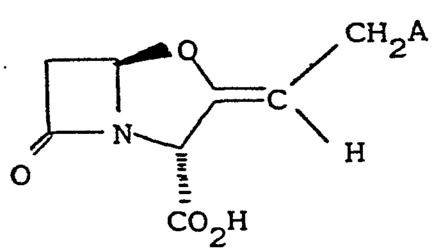

(VII)

wherein A is hydroxyl, substituted hydroxyl, thiol, substituted thiol, amino, mono- or di-hydrocarbyl-substituted amino, or mono- or di-acylamino.

A further advantageous composition comprises a compound of formula (I) or a pharamceutically acceptable salt or in vivo hydrolysable ester thereof together with a compound of formula (VIII) or a pharmaceutically acceptable salt or in vivo hydrolysable ester thereof:

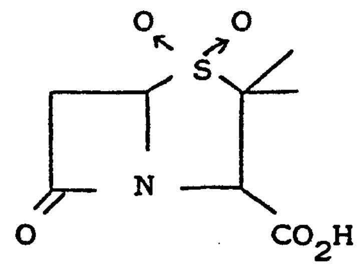

(VIII)

The present invention also provides a method of treating bacterial infections in animals, in particular humans or domestic mammals, which comprises the administration of the composition of this invention.

The following Examples illustrate the preparation of the compounds of this invention.

Example 1

Disodium 6,β-[R,S-2-(5-amino-3,4-dihydroxyphenyl)-2-sulphoacetamido]-6,α-methoxy penicillanate

a) 4-Hydroxy-3-methoxy-5-nitrophenylacetic acid

Homovanillic acid (1.82 g 10 mmol) was suspended in glacial acetic acid (15 ml) and a mixture of conc. nitric acid (0.5 ml) and glacial acetic acid (1 ml) added dropwise, maintaining a temperature of 10-15°C. After stirring for thirty minutes at room temperature the mixture was poured into iced water (100 ml) and the precipitated solid filtered off. This was dried to give the title compound (1.24 g, 55%), $\nu_{max}$ (Nujol), 1700 $cm^{-1}$; $\delta$ [$(CD_3)_2CO/(CD_3)_2SO$] 3.61 (2H, s, $CH_2CO$), 3.88 (3H, s, $OCH_3$), 7.19, 7.37 (2H, 2 x d, J, 3Hz, Ar).

b) Ethyl 4-hydroxy-3-methoxy-5-nitrophenylacetate

Ethyl homovanillate (21.0 g) in glacial acetic acid (150 ml) was treated, dropwise with conc. nitric acid (6.4 ml) in glacial acetic acid (7 ml) maintaining a temperature of 10-15°C. After stirring for thirty minutes this was poured into iced water (400 ml) and extracted with ethyl acetate (3 x 150 ml). The combined extracts were washed with saturated sodium bicarbonate (100 ml), water (100 ml), dilute hydrochloric acid (100 ml) and brine (100 ml). After drying with magnesium sulphate and evaporation, the residue was purified by chromatography on silica gel, eluting with 25% ethyl acetate in cyclohexane then crystallised from ethyl acetate/cyclohexane (11.94 g, 47%), mp 67-68°C; $\nu_{max}$ (Nujol) 1725, 1645, 1540 $cm^{-1}$;

δ ($CDCl_3$), 1.29 (3H, t, J 8Hz, $\underline{CH_3}CH_2$), 3.63 (2H, s, $CH_2CO$), 4.00 (3H, s, $OCH_3$), 4.22 (2H, q, J 8Hz, $\underline{CH_2}CH_3$), 7.21, 7.67 (2H, 2 x d J 2Hz, Ar), 10.81 (1H, bs, OH).

c) 3,4-Dihydroxy-5-nitrophenylacetic acid

i) 4-Hydroxy-3-methoxy-5-nitrophenylacetic acid (1.1g) in glacial acetic acid (4 ml) and 48% aqueous hydrobromic acid (3 ml) was refluxed, under nitrogen, for six hours. This was then poured into water (50 ml) and extracted with ethyl acetate (3 x 50 ml) which was dried over magnesium sulphate and evaporated to give the title compound (0.7g 68%).

ii) Ethyl 4-hydroxy-3-methoxy-5-nitrophenyl acetate (12.75g) in glacial acetic acid (50 ml) and 48% aqueous hydrobromic acid (75 ml) was refluxed for four hours while a stream of hydrogen bromide was passed through the mixture. After cooling the volume was reduced to 50 ml under vacuum and cooled. The solid was filtered off and dried (8.41 g 79%). The filtrate was extracted with ethyl acetate (3 x 50 ml) and the extracts dried and evaporated to give a further batch of the title compound (2.15g, 20%). $\nu_{max}$ (Nujol) 3370, 1690, 1540, 1350 $cm^{-1}$; δ [$(CD_3)_2CO$] 3.68 (2H, s, $CH_2$), 7.30, 7.60 (2H, 2 x d, J 2Hz, Ar), 9.71 (3H, bs, OH, $CO_2H$).

d) 3,4-Diacetoxy-5-nitrophenylacetic acid

3,4-Dihydroxy-5-nitrophenylacetic acid (4.26g) in dichloromethane (100 ml) was treated with triethylamine (8.4 ml, 3 eq) and 4-dimethylaminopyridine (0.24 g 0.1eq) then, after cooling in ice, acetic anhydride

(3.8 ml, 2eq) was added, dropwise. After stirring at room temperature for thirty minutes the solution was washed with dilute hydrochloric acid (50 ml) and water (50 ml), and, after drying over magnesium sulphate, was evaporated to a gum. This was crystallised from cyclohexane/ethyl acetate to give the title compound (4.51 g, 76%), mp 122-4°C; $\nu_{max}$ ($CHCl_3$) 3320, 1780, 1720, 1540, 1350 $cm^{-1}$; $\delta$ [$(CD_3)_2CO$], 2.35 (6H, s, $CH_3CO$), 3.88 (2H, s, $CH_2CO$), 7.67, 8.06, (2H, 2 x d J 2Hz, Ar), 8.82 (1H, bs, $CO_2H$).

e) <u>2-(3,4-Diacetoxy-5-nitrophenyl)-2-sulphoacetyl chloride</u>

3,4-Diacetoxy-5-nitrophenylacetic acid (2.97g 10 mmole) in dichloroethane (50 ml) was treated with oxalyl chloride (1.0 ml) and DMF (2 drops). After stirring at room temperature for one hour the clear solution was briefly degassed under vacuum. $\nu_{max}$ ($C_2H_4Cl_2$) 1790, 1540, 1360 $cm^{-1}$.

To this solution, at 0°, was added a suspension of sulphur trioxide/dioxan complex in dichloroethane (10 ml, 10 mmole). After allowing to come to room temperature this was stirred overnight. The solution so obtained was taken to contain 10 mmole of the title compound.

f) <u>Benzyl 6,β-[R,S-2-(3,4-diacetoxy-5-nitrophenyl)-2-sulphoacetamido]-6,α-methoxypenicillanate</u>

A solution of 2-(3,4-diacetoxy-5-nitrophenyl)-2-sulphoacetyl chloride (20 mmol) in dichloroethane (70 ml) was added dropwise to an ice-cold solution of benzyl 6,β-amino-6,α-methoxypenicillanate (6.72g 20 mmol) in dichloromethane (80 ml) containing triethylamine (10 ml). The solution was allowed to

warm to room temperature and, after stirring for thirty minutes, washed with dilute hydrochloric acid (50 ml) and water (70 ml). After drying over magnesium sulphate the solvent was evaporated. The product was isolated by chromatography on silica eluting with 5% methanol in chloroform. The fractions containing the required material were collected, evaporated and then passed down a column of Amberlite IR 120 ($Na^+$) ion-exchange resin in water. The water was evaporated to yield the title compound as its sodium salt (4.69g 33%). $\nu_{max}$ ($CHCl_3$), 1780, 1750, 1690, 1540 $cm^{-1}$ $\delta$ [$(CD_3)_2CO$] 1.26 (6H, m, $2xCH_3$), 2.23 (6H, s $OCOCH_3$), 3.43, 3.48 (3H, 2 x s, $OCH_3$), 4.33 (1H, bs, 3H), 4.96, 5.30 (1H, 2 x s, CHCO), 5.13 (2H, s, $\underline{CH_2}Ph$), 5.33 (1H, s, 5H), 7.31 (5H, s, Ph), 7.86, 8.34 (2H, 2 x s, Ar), 9.20, 9.41 (1H, 2 x bs, CONH).

g) <u>Disodium 6,β-[R,S-2-(5-amino-3,4-dihydroxyphenyl)-2-sulphoacetamido]-6,α-methoxypenicillanate</u>

Benzyl 6,β-[R,S-2-(3,4-dihydroxy-5-nitrophenyl)-2-sulphoacetamido]-6α-methoxypenicillanate (0.37g) was dissolved in water (60 ml) and hydrogenated for twenty minutes at atmospheric pressure and temperature with 10% palladium on charcoal (0.4g). After filtering under a protective atmosphere of carbon dioxide, the pH was adjusted to 5.5 with dilute sodium hydroxide and the solution freeze-dried to yield the title compound (0.28g, 90%). $\nu_{max}$ (KBr), 1760, 1670, 1610 $cm^{-1}$; $\delta$ [$(CD_3)_2SO$], 1.39 (6H, bs, $2xCH_3$), 3.23 (3H, s, $OCH_3$), 3.94 (1H, s, 3H), 4.16, 4.38 (1H, 2 x s, CHCO), 5.28 (1H, s, 5H), 6.20, 6.29 (2H, 2 x s, Ar), 9.33 (1H, s, CONH). Antibacterial activities (MIC µg/ml); E.Coli ESS, 5.0; E.Coli JT4, 25; Ps aeruginosa 10662, 50; Ps aeruginosa Dalgleish $10^{-2}$, 50; N catarrhalis 1502, <0.2.

Example 2

Disodium 6,β-[R,S-2-(3,4-dihydroxy-5-nitrophenyl)-2-sulphoacetamido]-6,α-methoxypenicillanate

a) Benzyl 6,β-[R,S-2-(3,4-dihydroxy-5-nitrophenyl)-2-sulphoacetamido]-6,α-methoxypenicillanate

Benzyl 6,β-[R,S-2-(3,4-diacetoxy-5-nitrophenyl)-2-sulphoacetamido]-6,α-methoxypenicillanate (1.44 g) was dissolved in water (74 ml) and acetyl esterase (E.C. 3.1.1.6, 24 units) added. The pH was adjusted to 8.7 and maintained there for 10 minutes. After adjusting to pH 6.0 the solution was extracted with ethyl acetate (50 ml) then n-butanol (3 x 50 ml). The combined butanol extracts were washed with saturated brine (50 ml), dried over magnesium sulphate and evaporated. The residue was dissolved in water (20 ml) and passed down a column of Amberlyst XAD-2 resin, eluting with 50% aqueous methanol. The appropriate fractions were evaporated to give the title compound as its sodium salt (0.4 g 31%). $\nu_{max}$ (KBr) 1770, 1740, 1680, 1620 $cm^{-1}$, δ [$(CD_3)_2SO$] 1.25, 1.38 (6H, 2xs, $2x2CH_3$), 3.43 (3H, s, $OCH_3$) 4.50 (1H, s, 3H), 4.73 (1H, s, CHCO) 5.21 (2H, s, $CH_2Ph$), 5.35 (1H, s, 5H), 7.42 (7H, bs, Ar), 9.49 (1H, bs, CONH).

b) Disodium 6,β-[R,S-2-(3,4-dihydroxy-5-nitrophenyl)-2-sulphoacetamido]-6,α-methoxypenicillanate

Benzyl 6,β-[R,S-2-(3,4-dihydroxy-5-nitrophenyl)-2-sulphoacetamido]-6,α-methoxypenicillanate (0.8 g) in water (50 ml) was adjusted to pH 10.0 and maintained at that pH for four hours. The pH was then adjusted to 6.0 by addition of Amberlyst IR 120 ($H^+$) ion-exchange resin. The solution was filtered and, after concentration, the title compound was isolated by

chromatography on Amberlyst XAD-2 resin, eluting with water (45 mg, 7%). $\nu_{max}$ (KBr), 1760, 1675, 1610, 1540, 1350 $cm^{-1}$, $\delta$ ($D_2O$), 1.18, 1.39 (6H, 2xs, $2x2CH_3$), 3.46, 3.59 (3H, 2xs, $OCH_3$), 4.22 (1H, s, 3H), 4.98, 5.16 (1H, 2xs, CHCO), 5.55 (1H, s, 5H) 7.27, 7.76 (2H, 2xd, J 2Hz, Ar). Antibacterial activities (MIC μg/ml): E.Coli ESS, 0.2; E.Coli JT4, 2.5; Ps aeruginosa 10662, 5.0; Ps aeruginosa Dalgleish $10^{-2}$, 1.0; N catarrhalis 1502, <0.02.

Example 3

Disodium 6,β-[R-2-(3,4-dihydroxy-5-nitrophenyl)-2-sulphoacetamido]-6,α-methoxypenicillanate

a) Benzyl 6,β-[R-2-(3,4-diacetoxy-5-nitrophenyl)-2-sulphoacetamido]-6,α-methoxypenicillanate

To an ice cold solution of benzyl 6,β-amino-6,α-methoxypenicillanate (3.36 g, 10 mmol) and N-methylmorpholine (3.85 ml, 35 mmole) in dichloromethane (50 ml) was added, dropwise, a solution of 2-(3,4-diacetoxy-5-nitrophenyl)-2-sulphoacetyl chloride (10 mmol) in dichloroethane (50 ml). The solution was allowed to come to room temperature and, after stirring for thirty minutes, washed with dilute hydrochloric acid (50 ml) and water (50 ml). After drying over magnesium sulphate the solvent was evaporated.

The residue was purified by chromatography on silica gel, eluting with 5% methanol in chloroform. The fractions containing the required compound were evaporated and crystallised from 4-methyl-pentan-2-one. The title compound was obtained as its N-methylmorpholine salt in 9.7% yield. $\nu_{max}$ (KBr), 1780, 1745, 1690, 1540, 1375, 1350 $cm^{-1}$; δ [$(CD_3)_2SO$] 1.23, 1.30 (6H, 2xs, $2xCH_3$), 2.33 (6H, s, $OCOCH_3$), 2.78 (3H, s, $N-CH_3$), 3.20, 3.78 (9H, 2xbs, morpholine), 3.44 (3H, s, $OCH_3$), 4.47 (1H, s, 3H) 5.10 (1H, s, CH.CO) 5.17 (2H, s, $\underline{CH_2}Ph$), 5.32 (1H, s, 5H), 7.37 (5H, s, Ph), 7.75, 8.20 (2H, 2xd, J, 2Hz, Ar), 9.55 (1H, s, CONH).

b) Disodium 6,β-[R-2-(3,4-dihydroxy-5-nitrophenyl)-2-sulphoacetamido]-6,α-methoxypenicillanate

Benzyl 6,β-[R-2-(3,4-diacetoxy-5-nitrophenyl)-2-sulphoacetamido]-6,α-methoxypenicillanate, N-methylmorpholine salt (0.4 g) was dissolved in water (5 ml)

and the solution was added to a suspension of Alcalase (Subtilisin Carlsberg) bound to Mitsubishi WK10S resin (8.0 g) in water (10 ml) at pH 7.5 and 37°C. The stirred suspension was maintained at these conditions for 5 hours then filtered and adjusted to pH 6.0 with dilute acetic acid. The solvent was evaporated and the title compound isolated by chromatography on Amberlyst XAD-2 resin then Sephadex G25-F eluting with water (106 mg 40%). δ ($D_2O$), 1.18, 1.39 (6H, 2xs, 2x2$CH_3$), 3.58 (3H, s, $OCH_3$), 4.20 (1H, s, 3H), 4.98 (1H, s, CHCO), 5.60 (1H, s, 5H), 7.26, 7.74 (2H, 2xd, J 3Hz, Ar).

Example 4

Disodium 6,β-[R-2-(3-chloro-4,5-dihydroxyphenyl)-2-sulphoacetamido]-6,α-methoxypenicillanate

a) 3-Chloro-4-hydroxy-5-methoxyphenylacetic acid

Ethyl 4-hydroxy-3-methoxy-5-nitrophenylacetate (10.4 g) with 1:1 ethanol:THF (120 ml) was hydrogenated in the presence of 10% palladium on carbon (1.0 g) for 1.5 hours. The mixture was filtered and the filtrate evaporated to dryness. The residue was dissolved in 50% hydrochloric acid (160 ml), cooled to 0°C and treated dropwise with sodium nitrite (3.04 g) in water (40 ml). Cupic chloride (4.0 g) in concentrated hydrochloric acid (40 ml) was then added and the mixture heated at 45°C for 3.5 hours, allowed to cool, then extracted with ethyl acetate (3 x 200 ml). The extracts were washed with water (2 x 100 ml) and brine (100 ml), dried and evaporated to a gum, 7.93 g, δ[$(CD_3)_2CO$] 3.59 (2H, s, $CH_2$), 3.90 (3H, s, $OCH_3$), 6.97 (2H, s, Ar), 8.10 (2H, s, OH, $CO_2H$).

b) 3-Chloro-4,5-dihydroxyphenylacetic acid

3-chloro-4-hydroxy-5-methoxyphenylacetic acid (7.93 g) was suspended in glacial acetic acid (100 ml) and 48% hydrobromic acid (100 ml) and heated under reflux for two hours. The mixture was poured into water (300 ml) and extracted with ethyl acetate (3 x 100 ml). The extracts washed with water (3 x 100 ml), dried and evaporated to an oil which was crystallised from ethyl acetate-cyclohexane, 3.52 g, m.p. 164-165°C, δ[$(CD_3)_2CO$] 3.53 ($2H_2$, s, $CH_2$), 6.83 (2H, s, Ar), 7.53 (3H, s, 2 x OH, $CO_2H$).

0091302

c) 3-Chloro-4,5-diacetoxyphenylacetic acid

3-Chloro-4,5-dihydroxyphenylacetic acid (3.03 g) in dichloromethane (25 ml) was treated with triethylamine (6.3 ml) and 4-dimethylaminopyridine (180 mg) then with acetic anhydride (2.85 ml). The reaction was stirred for 10 minutes, washed with dilute hydrochoric acid, dried, evaporated to an oil which was crystallised from chloroform-carbon tetrachloride, 3.45 g, m.p. 142-143°C, $\delta(CDCl_3)$, 2.30, 2.35 (6H, 2 x s, 2 x $OCOCH_3$), 3.63 (2H, s, $CH_2$), 7.18 and 7.37 (2H, 2 x s, J 2Hz, Ar), 10.13 (1H, s, $CO_2H$).

d) Benzyl 6,β-[2-(3-chloro-4,5-diacetoxyphenyl)-2-sulphoacetamido]-6,α-methoxypenicillanate triethylammonium salt

3-Chloro-4,5-diacetoxyphenylacetic acid (1.43 g, 5 mmol) in dichloromethane (50 ml) was treated with oxalyl chloride (0.5 ml) and DHF (1 drop), stirred for 30 minutes and evaporated to dryness. The residue was dissolved in dichloromethane, sulphur trioxide-dioxan complex in 1,2-dichloromethane (5 ml of 1 molar solution) added and the solution stirred overnight then added dropwise to benzyl 6,β-amino-6,α-methoxy-pencillanate (1.63 g, 5 mmol) and triethylamine (2.5 ml) in dichloromethane (25 ml) cooled in an ice bath. After 30 minutes the mixture was washed with dilute hydrochoric acid, then water, dried and evaporated to a gum which was chromatographed on silica eluting with 5% methanol in chloroform to give benzyl 6,β-[R,S-2-(3-chloro-4,5-diacetoxyphenyl)-2-sulphoacetamido]-6,α-methoxy penicillanate triethylamine salt, 1.63 g, followed by benzyl 6,β-[R-2-(3-chloro-4,5-diacetoxyphenyl)-2-sulphoacetamido] 6,α-methoxypenicillanate triethylammonium salt, 0.62 g, $\nu_{max}$ ($CHCl_3$) 1790, 1775, 1710, 1690, 1480, 1370, 1175

and 1030 $cm^{-1}$, δ [$(CD_3)_2CO$] 0.9-1.5 (15H, m, 2 x $2CH_3$, $HN(CH_2\underline{CH_3})_3$), 2.32, 2.37 (6H, 2 x s, 2 x $OCOCH_3$), 3.17 (6H, q, J7Hz, $HN(\underline{CH_2}CH_3)_3$), 3.63 (3H, s, $OCH_3$), 4.55 (1H, s, 3H), 5.32 (2H, s, $O\underline{CH_2}Ph$), 5.42 (1H, s, <u>CH</u>CONH), 5.55 (1H, s, 5H), 7.53 (5H, s, $CH_2\underline{Ph}$), 7.70, 7.98 (2H, 2 x d, J2Hz, Ar), 9.43 (1H, s, CONH).

e) <u>Disodium 6,β-[R-2-(3-chloro-4,5-diacetoxyphenyl)-2-sulphoacetamido]-6,α-methoxypenicillanate</u>

Benzyl 6,β-[R-2-(3-chloro-4,5-diacetoxyphenyl)-2-sulphoacetamido]-6,α-methoxypenicillanate triethylammonium salt (0.62 g) in water (50 ml) was hydrogenated in the presence of 10% palladium on carbon (0.6 g) for 1 hour. The mixture was filtered, passed through a column of 'Amberlite' IR120 ($Na^+$) resin in water and the eluant freeze dried, 0.40 g, $\nu_{max}$ (KBr) 1770, 1685, 1608, 1482, 1372, 1210 and 1042 $cm^{-1}$, δ [$(CD_3)_2SO$] 1.28, 1.32 (6H, 2 x s, 2 x 2 $CH_3$), 2.30 (6H, s, 2 x $OCOCH_3$), 3.40 (3H, s, $OCH_3$), 3.89 (1H, s, 3H), 4.87 (1H, s, <u>CH</u>CONH), 5.25 (1H, s, 5H), 7.28 and 7.58 (2H, 2 x broads, Ar), 9.27 (1H, s, CONH).

f) <u>Disodium 6,β-[R-2-(3-chloro-4,5-dihydroxyphenyl)-2-sulphoacetamido]-6,α-methoxypenicillanate</u>

Disodium 6,β-[R-2-(3-chloro-4,5-diacetoxyphenyl)-2-sulphoacetamido]-6,α-methoxypenicillanate (260 mg) in water (10 ml) was adjusted to pH 7.5 and 37°C then treated with protease type VIII (subtilisin Carlsberg ex. Sigma Chemical Co. Ltd.) (30 mg). pH7.5 was maintained for 2 hours then lowered to pH 6.5 and the reaction mixture chromatographed on Amberlite XAD-2 eluting with water. The fractions containing the product (h.p.l.c. analysis) were combined and freeze dried, 148 mg $\nu_{max}$ (KBr) 1765, 1685, 1610, 1240, 1205,

1097 and 1042 $cm^{-1}$, δ $[(CD_3)_2SO]$ 1.33 (6H, s, 2 x $2CH_3$), 3.35 (3H, s, $OCH_3$), 3.85 (1H, s, 3H), 4.44 (1H, s, CHCONH), 5.23 (1H, s, 5H). 6.81 (2H, s, phenyl 2H and 6H), 9.23 (1H, s, CONH).

Example 5

Disodium 6,ß-[R-2-(2-chloro-4,5-diacetoxyphenyl)-2-sulphoacetamido]-6,α-methoxypenicillanate

a) 2-Chloro-4,5-dihydroxyphenylacetic acid

3,4-Dihydroxyphenylacetic acid (0.79 g) in glacial acetic acid (10 ml) was treated with sulphuryl chloride (0.4 ml), stirred for two hours, poured into water (75 ml) and extracted with ethyl acetate (3 x 50 ml). The extracts were washed with water (4 x 75 ml) and brine (75 ml), dried and evaporated to a solid which was crystallised from ethyl acetate-cyclohexane, 0.90 g, m.p. 180-181°C, δ$[(CD_3)_2CO]$ 3.37 (2H, s, $CH_2$), 6.90 (2H, s, Ar), 9.27 (3H, s, 2 x OH, $CO_2H$).

b) 2-Chloro-4,-5-diacetoxyphenylacetic acid

2-Chloro-4,5-dihydroxyphenylacetic acid was treated as in example 4c to give the title compound as an oil, δ($CDCl_3$) 2.17 (6H, s, 2 x $OCOCH_3$), 3.73 (2H, s, $CH_2$), 7.17, 7.26 (2H, 2 x s, Ar), 10.92 (1H, s, $CO_2H$).

c) Benzyl 6,ß-[2-(2-chloro-4,5-diacetoxyphenyl)-sulphoacetamido]-6,α-methoxypenicillanate triethylammonium salt

2-Chloro-4,5-diacetoxyphenylacetic acid (2.86 g) was treated as described in example 4d. Two fractions were obtained from the chromatography of the product, benzyl 6,ß-[R,S-2-(2-chloro-4,5-diacetoxyphenyl)-2-sulphoacetamido]-6,α-methoxypenicillanate

triethylammonium salt, 0.48 g, followed by benzyl 6,β-[R-2-(2-chloro-4,5-diacetoxyphenyl)-2-sulphoacetamido]-6,α-methoxypenicillanate triethylammonium salt, 0.38 g, δ[$(CD_3)_2CO$] 1.1 - 1.6 (15H, m, 2 x $2CH_3$, HN $(CH_2\underline{CH_3})_3$), 2.29, 2.30 (6H, 2 x s, 2 x $OCOCH_3$), 3.13 (6H, q, J 7Hz, $HN(CH_2CH_3)_3$), 3.58 (3H, s, $OCH_3$), 4.53(1H, s, 3H), 5.27 (2H, s, $O\underline{CH_2}Ph$), 5.48 (1H, s, <u>CH</u>CONH), 5.57 (1H, s, 5H), 7.3-7.7 (7H, m, Ph, Ar), 9.43 (1H, s, CONH).

d) <u>Disodium 6,β-[R-2-(2-chloro-4,5-diacetoxyphenyl)-2-sulphoacetamido]-6,α-methoxypenicillanate</u>

Benzyl 6,β-[R-2-(2-chloro-4,5-diacetoxyphenyl)-2-sulphoacetamido]-6,α-methoxypenicillanate triethylammonium salt (0.38 g) in water (25 ml) was hydrogenated in the presence of 10% palladium on carbon (0.38 g) for 1 hour. The mixture was filtered, the filtrate passed through a column of 'Amberlite' IR120($Na^+$) in water and the eluant freeze dried, 0.17 g $\nu_{max}$ (KBr) 1765, 1685, 1610, 1490, 1370, 1200, 1140, and 1040$cm^{-1}$, δ[$(CD_3)_2SO$] 1.35 (6H, s, 2 x $2CH_3$), 2.24, 2.25 (6H, 2 x s, 2 x $OCOCH_3$), 3.40 (3H, s, $OCH_3$). 3.95 (1H, s, 3H). 5.21 (1H, s, <u>CH</u>CONH), 5.27 (1H, s, 5H) 7.38, 7.68 (2H, 2 x s, Ar), 9.40 (1H, s, CONH).

Example 6

Disodium 6,β-[2-(4,5-diacetoxy-2-methylphenyl)-2-sulpho acetamido]-6,α-methoxypenicillanate

i) 4,5-Dimethoxy-2-methylphenylthio acetomorpholide

A mixture of 4,5-dimethoxy-2-methylacetophenone (32.72 g), morpholine (45 ml) and sulphur (16.9 g) was refluxed for 2 hours, allowed to cool to room temperature, diluted with chloroform (200 ml), washed with water (2 x 200ml), 5N hydrochloric acid (200 ml), water (3 x 200 ml), dried over magnesium sulphate, filtered and evaporated to dryness. Crystallisation from ethanol-water gave 29.15 g, 59% δ($CDCl_3$) 2.20 (3H, s, Ar$CH_3$), 3.50 (2H, s, $CH_2CS$), 3.5-4.45 (8H, m, -N   O), 3.83 (6H, s, 2 x $OCH_3$), 6.68, 6.82 (2H, 2 x s, Ar).

ii) 4,5-Dimethoxy-2-methylphenylacetic acid

A mixture of 4,5-dimethoxy-2-methylphenylthioaceto-morpholide (28 g) and sodium hydroxide (28 g) in water (300 ml) was refluxed for 18 hours. The pH was reduced to 10 by additions of dry ice and the mixture washed with ethyl acetate (2 x 200 ml). The aqueous phase was acidified to pH 2 with concentrated hydrochloric acid and extracted with ethyl acetate (2 x 200 ml). The combined extracts were washed with water (3 x 200 ml) dried over magnesium sulphate, filtered and evaporated to dryness. Chromatography on silica eluting with chloroform grading to 2% methanol in chloroform gave 12.77 g, 60.8%. δ($CDCl_3$) 2.25 (3H, s, $ArCH_3$), 3.61 (2H, s, $CH_2$), 3.86 (6H, s, 2 x $OCH_3$), 6.70, 6.73 (2H, 2 x s, Ar), 11.20 (1H, bs, $CO_2H$).

iii) 4,5-Dihydroxy-2-methylphenylacetic acid

A solution of 4,5-dimethoxy-2-methylphenylacetic acid (11.5 g) in glacial acetic acid (130 ml) and 48% hydrobromic acid (130 ml) was refluxed for 3 hours. Allowed to cool to room temperature, then water (200 ml) and ethyl acetate (300 ml) added. The pH was adjusted to 3.0 by additions of solid sodium carbonate. The aqueous phase was separated and extracted with ethyl acetate (2 x 200 ml). The combined ethyl acetate phases were washed with water (3 x 200 ml), saturated brine (200 ml), dried over magnesium sulphate, filtered and evaporated to dryness *in vacuo* to yield 7.36 g, 73.8% $\delta[(CD_3)_2CO]$ 2.11 (3H, s, $ArCH_3$), 3.47 (2H, s, $CH_2CO$), 6.61, 6.69 (2H, 2 x s, Ar), 8.48 (3H, bs, 2 x OH, $CO_2H$).

iv) 4,5-Diacetoxy-2-methylphenylacetic acid

A solution of 4,5-dihydroxy-2-methylphenylacetic acid (7.36g, 40mmol ) in 40% aqueous-THF (150ml) at pH 7.5 with saturated sodium carbonate solution, and room temperature, was treated with acetic anhydride (9.5ml, 90mmol) After 45 minutes and after a further 15 minutes portions of acetic anhydride (4.75ml, 45 mmol) were added. The reaction was kept at pH 6.5 throughout by addition of saturated sodium carbonate solution. The mixture was washed with ether (3 x 250ml), layered with ethyl acetate (200 ml), acidified to pH 2.5 with 5N hydrochloric acid and the aqueous discarded. The ethyl acetate phase was washed with water (3x250ml), dried over magnesium sulphate, filtered and evaporated to dryness in vacuo to yield 8.2g, 76.3% $\nu_{max}$ $(CHCl_3)$ 3050 (br), 1760, 1710, 1500, 1370, 1285 and 1180 $cm^{-1}$, $\delta[(CD_3)_2CO]$ 2.20 (6H, s, 2 x $CH_3CO$), 2.27 (3H, s, Ar$CH_3$), 3.60 (2H, s, $CH_2$), 6.94 6.96 (2H, 2xs, Ar).

v) Benzyl 6,β-[2-(4,5-diacetoxy-2-methylphenyl)-2-sulphoacetamido]-6,α-methoxypenicillanate triethylammonium salt

Prepared as described in example 4 iv except that 4,5-diacetoxy-2-methylphenylacetic acid (2.66g, 10 mmol) was used to yield a gum, which was chromatographed on silica gel eluting with 5% methanol in chloroform to give benzyl 6,β-[S-2-(4,5-diacetoxy-2-methylphenyl)-2-sulphoacetamido]-6,α-methoxypenicillanate triethylammonium salt, 0.77g, 10.1%, $\nu_{max}$ $(CHCl_3)$ 3000, 1780, 1710, 1500, 1370 and 1190 (br)$cm^{-1}$, $\delta[(CD_3)_2CO]$ 1.18 [9H, t, J 7Hz, $\overset{\oplus}{N}H(CH_2CH_3)_3$], 1.33, 1.53 (6H, 2xs, $2xCH_3$), 2.23 (6H, s, $2xCH_3CO$), 2.48 (3H, s, Ar$CH_3$), 2.98 [6H, q, J 7Hz, $H\overset{\oplus}{N}(CH_2CH_3)_3$], 3.52 (3H, s, $OCH_3$), 4.52 (1H, s, 3H), 5.02 (1H, s, CHCON), 5.23 (2H, s, $OCH_2Ph$), 5.50 (1H, s, 5H),

7.00-7.75 (7H, m, Ph, Ar), 9.47 (1H, s, CONH), followed by benzyl 6,β-[R-2-(4,5-diacetoxy-2-methylphenyl)-2-sulphoacetamido]-6,α-methoxypenicillanate triethylammonium salt, 0.68 g, 8.9%, $\nu_{max}$ ($CHCl_3$) 3000, 1780, 1710, 1500, 1370 and 1200 (b )$cm^{-1}$, δ[$(CD_3)_2CO$] 1.17 [9H, t, J 7Hz, $H\overset{\oplus}{N}(CH_2\underline{CH_3})_3$], 1.31, 1.42 (6H, 2xs, $2x2CH_3$), 2.28 (6H, s, $2xCH_3CO$), 2.44 (3H, s, $Ar\underline{CH_3}$), 3.00 [6H, q, J 7Hz, $H\overset{\oplus}{N}(\underline{CH_2}CH_3)_3$], 3.53 (3H, s, $OCH_3$), 4.50 (1H, s, 3H), 5.21 (2H, s, $O\underline{CH_2}Ph$), 5.30 (1H, s, CHCON), 5.46 (1H, s, 5H), 7.01-7.80 (7H, m, Ph, Ar), 9.31 (1H, s, CONH).

vi) Disodium 6,β-[R-2-(4,5-diacetoxy-2-methylphenyl)-2-sulphoacetamido]-6,α-methoxypenicillanate

Prepared as described in example 4 v except that benzyl 6,β-[R-2-(4,5-diacetoxy-2-methylphenyl)-2-sulphoacetamido]-6,α-methoxypenicillanate triethylammonium salt (0.68g) was used, and the mixture passed through a column of Dowex 50W-8X resin before being freeze dried to yield 0.4g, 72.7%, $\nu_{max}$ (KBr) 3450 (br), 1765, 1680, 1615, 1500, 1370 and 1210 $cm^{-1}$, δ ($D_2O$) 1.23, 1.39 (6H, 2xs, $2x2CH_3$), 2.37, 2.48 (9H, 2xs, $2xCH_3CO$, $Ar\underline{CH_3}$), 3.61 (3H, s, $OCH_3$), 4.25 (1H, s, 3H), 5.42, 5.54 (2H, 2xs, CHCON, 5H), 7.20, 7.84 (2H, 2xs, Ar).

vii) Disodium 6,β-[S-2-(4,5-diacetoxy-2-methylphenyl)-2-sulphoacetamido]-6,α-methoxypenicillanate

Prepared as described in example 4 v except that benzyl 6,β-[S-2-(4,5-diacetoxy-2-methylphenyl)-2-sulphoacetamido]-6,α-methoxypenicillanate triethylammonium salt (0.77g) was used, and the mixture passed through a column of Dowex 50W-8X resin being freeze dried to yield 0.3g, 48%, $\nu_{max}$ (KBr) 3450 (br), 1765, 1680, 1615, 1500, 1370 and 1210 (br)$cm^{-1}$, δ($D_2O$) 1.38, 1.48 (6H, 2xs, $2x2CH_3$), 2.33 (6H, s, $2xCH_3CO$), 2.48 (3H, s, $Ar\underline{CH_3}$), 3.45 (3H, s, $OCH_3$), 4.33 (1H, s, 3H), 5.38, 5.54 (2H, 2xs, CHCON, 5H), 7.21, 7.69 (2H, 2xs, Ar).

Example 7

Disodium 6,β-[2-(4,5-diacetoxy-3-methylphenyl)-2-sulpho-acetamido -6,α-methoxypenicillanate

i) 4,5-Dimethoxy-3-methylphenylthioacetomorpholide

A mixture of 4,5-dimethoxy-3-methylacetophenone (27.66g), morpholine (38 ml) and sulphur (14.3g) was refluxed for 2 hours, allowed to cool to room temperature, diluted with chloroform (200ml), 5N hydrochloric acid (200ml), water (3 x 200ml), dried over magnesium sulphate, filtered and evaporated to dryness to yield 40.3g, 96%. $\delta(CDCl_3)$ 2.26 (3H, s, Ar$\underline{CH_3}$), 3.28-4.5 (16H, m, 2 x $CH_3O$, $CH_2\overset{\overset{S}{\|}}{C}$, 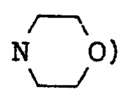), 6.62, 6.80 (2H, 2 x s, Ar).

ii) 4,5-Dimethoxy-3-methylphenylacetic acid

Prepared by the method used in example 6 ii except that 4,5-dimethoxy-3-methylphenylthioacetomorpholide (40g), sodium hydroxide (40g) and water (400ml) were used to yield 16.5g, 58%. $\nu_{max}$ $(CHCl_3)$ 2930, 1710, 1590, 1490, 1150, 1095 and 1005 $cm^{-1}$. $\delta[(CD_3)_2CO]$ 2.21 (3H, s, Ar$\underline{CH_3}$), 3.50 (2H, s, $CH_2CO$), 3.73, 3.80 (6H, 2 x s, 2 x $OCH_3$), 6.68, 6.81 (2H, 2 x d, J 2Hz, Ar), 10.00 (1H, bs, $CO_2H$).

iii) 4,5-Dihydroxy-3-methylphenylacetic acid

A solution of 4,5-dimethoxy-3-methylphenylacetic acid (16.5 g) in glacial acetic acid (200ml) and 48% hydrobromic acid (200ml) was refluxed for 5 hours. Hydrogen bromide gas was bubbled through the reaction for the final hour. Allowed to cool to room temperature,

water (300ml) and ethylacetate (400ml) added. The pH was adjusted to 3.0 by additions of solid sodium carbonate. The aqueous layer was separated and extracted with ethyl acetate (2 x 400ml). The combined ethyl acetate phases were washed with water (3 x 400ml), brine (300ml), dried over magnesium sulphate, filtered and evaporated to dryness, chromatographed on silica gel eluting with 10% methanol in chloroform to yield 5.67g, 39.7%. $\delta$ [$(CD_3)_2CO$] 2.15 (3H, s, Ar$\underline{CH_3}$), 3.37 (2H, s, $CH_2$), 6.49, 6.61 (2H, 2 x d, J = 2Hz, Ar), 7.53 (3H, bs, 2 x OH, $CO_2H$).

iv) 4,5-Diacetoxy-3-methylphenylacetic acid

Prepared by the method described in example 6 iv except that 4,5-dihydroxy-3-methylphenylacetic acid (4.39g, 24 mmol) was used to give 4.66g, 73% $\nu_{max}$ ($CHCl_3$) 3050 (br), 1765, 1715, 1370, 1180 and 1135$cm^{-1}$. $\delta$[$(CD_3)_2CO$] 2.11, 2.17, 2.21 (9H, 3 x s, 3 x $CH_3$), 3.56 (2H, s, $CH_2$), 6.93 (2H, m, Ar), 10.15 (1H, bs, $CO_2H$).

v) Benzyl 6,β-[2-(4,5-dioacetoxy-3-methylphenyl)-2-sulphoacetamido]-6,α-methoxypenicillanate

Prepared as described in example 4 iv except that 4,5-diacetoxy-3-methylphenylacetic acid (2.66g, 10 mmol) was used to yield a gum which was chromatographed on silica gel eluting with 5% methanol in chloroform to give benzyl 6,β-αS-2-(4,5-diacetoxy-3-methylphenyl)-2-sulphoacetamido]-6,α-methoxypenicillanate triethylammonium salt, 0.62g, 8.1%, $\nu_{max}$ ($CHCl_3$) 2290, 1770 (br), 1690, 1370 and 1220 (b)$cm^{-1}$, $\delta$[$(CD_3)_2CO$] 1.15 [9H, t, J 7Hz, $\overset{\oplus}{H}N(CH_2\underline{CH_3})_3$], 2.18, 2.24, 2.26 (9H, 3 x s, 2 x $CH_3CO$, Ar$\underline{CH_3}$), 3.00 [6H, q, J 7Hz, $\overset{\oplus}{H}N(\underline{CH_2}CH_3)_3$], 3.52 (3H, s, $OCH_3$), 4.53 (1H, s, 3H), 4.82 (1H, s, 5H), 5.23 (2H, s, -O$\underline{CH_2}$Ph), 5.50 (1H, s, 5H), 7.40 (7H, m, Ph, Ar), 9.35 (1H, s, CONH) followed by benzyl 6,β-[R-2-(4,5-diacetoxy-3-methylphenyl)-2-

sulphoacetamido]-6,α-methoxypenicillanate 0.7g, 9.2%, $\nu_{max}$ ($CHCl_3$) 2995, 1770 (br), 1695, 1370 and 1210 (br)$cm^{-1}$, δ [$(CD_3)_2CO$] 1.17 [9H, t, J 7Hz, $\overset{\oplus}{H}N(CH_2\underline{CH_3})_3$], 1.28, 1.33 (6H, 2 x s, 2 x $2CH_3$), 2.13, 2.25, 2.27 (9H, 3xs, $2xCH_3CO$, $Ar\underline{CH_3}$), 3.00 [6H, q, J 7Hz, $\overset{\oplus}{N}H(\underline{CH_2}CH_3)_3$] 3.52 (3H, s, $OCH_3$), 4.47 (1H, s, 3H), 5.21 (3H, s, $O\underline{CH_2}Ph$, CHCON), 5.48 (1H, s, 5H), 7.40 (7H, m, Ph, Ar), 9.24 (1H, s, CONH).

vi) Disodium 6,β-[R-2-(4,5-diacetoxy-3-methylphenyl)-2-sulphoacetamido]-6,α-methoxypenicillanate

Prepared as described in example 4 v except that benzyl 6,β-[R-2-(4,5-diacetoxy-3-methylphenyl)-2-sulphoacetamido]-6,α-methoxypenicillanate (0.7g) was used, and the mixture passed through a column of Dowex 50W-8X before being freeze-dried. 0.3g, 53%, $\nu_{max}$ (KBr) 3450 (br), 1760, 1680, 1610 and 1210 (br)$cm^{-1}$, δ($D_2O$) 1.18, 1.41 (6H, 2 x s, 2 x $2CH_3$), 2.24, 2.36, 2.40 (9H, 3 x s, 2 x $CH_3CO$, $Ar\underline{CH_3}$), 3.60 (3H, s, $OCH_3$), 4.23 (1H, s, 3H), 5.12 (1H, s, CHCON), 5.53 (1H, s, 5H), 7.42, 7.52 (2H, 2 x d, J 2Hz, Ar).

Example 8

Disodium 6,β-[2-(4,5-diacetoxy-2,3-dichlorophenyl)-2-sulphoacetamido]-6,α-methoxypenicillanate

i) Ethyl 2,3-dichloro-4-hydroxy-5-methoxyphenylacetate

Ethyl homovallinate (10.50g) in glacial acetic acid (100ml) was cooled in an ice bath and treated dropwise with sulphuryl chloride (8ml). When the addition was complete the mixture was stirred for a further 30 minutes. Then it was poured onto ice and the off-white solid collected, washed with water and dried *in vacuo*, 11.38g, m.p. 134-136$^{\circ}$ C from ethyl acetate-cyclohexane, $\delta[(CD_3)_2CO]$ 1.27 (3H, t, J 7Hz), 3.73 (2H, s), 3.88 (3H, s), 4.17 (2H, q, J7Hz), 6.89 (1H, s), 8.03 (1H, bs).

ii) 2,3-Dichloro-4,5-dihydroxyphenylacetic acid

Ethyl 2,3-dichloro-4-hydroxy-5-methoxyphenylacetate (13.33g) in glacial acetic acid (100ml) and 48% hydrobromic acid (100ml) were heated under reflux for 5 hours then for a further hour with a stream of hydrogen bromide gas bubbling through the solution. The mixture was allowed to cool then poured into iced water, adjusted to pH3 with solid sodium bicarbonate and extracted with ethyl acetate (3x150ml). The combined extracts were washed with water (3x200ml), dried and evaporated to a solid which was crystallised from ethyl acetate-cyclohexane, 4.29g, 37%, m.p. 179-181$^{\circ}$C, $\delta[(CD_3)_2CO]$ 3.70 (2H,s), 6.87 (1H, s), 8.00 (3H, bs).

iii) 4,5-Diacetoxy-2,3-dichlorophenylacetic acid

Acetic anhydride (3.8ml) was added dropwise to a mixture of 2,3-dichloro-4,5-dihydroxyphenylacetic acid

(4.76g), triethylamine (8.4ml) and 4-dimethylaminopyridine (240mg) in dichloromethane (100ml). The reaction mixture was stirred for 30 minutes then washed with dilute hydrochloric acid (100ml) and water (2x100ml), dried and evaporated to a solid which was crystallised from ethyl acetate-cyclohexane, 5.07g, 79%, $\delta[(CD_3)_2CO]$ 2.31 (6H, s), 3.82 (2H, s), 7.23 (1H, s).

iv) Benzyl 6,β-[2-(4,5-diacetoxy-2,3-dichlorophenyl)-2-sulphoacetamido]-6,α-methoxypenicillanate triethylammonium salt

Prepared as described in example 4 iv except that 4,5-di-acetoxy-2,3-dichlorophenylacetic acid (3.21g, 10mmole) was used to yield a gum which was chromatographed on silica gel eluting with 5% methanol in chloroform to give benzyl 6,β-[R,S-2-(4,5-diacetoxy-2,3-dichlorophenyl)-2-sulphoacetamido]-6,α-methoxypenicillanate triethylammonium salt, 1.93g, 23.5% followed by benzyl 6,β-[R-2-(4,5-diacetoxy-2,3-dichlorophenyl)-2-sulphoacetamido]-6,α-methoxypenicillanate, 0.56g, 6.8 $\nu_{max}$ $(CHCl_3)$ 2995, 1780, 1690, 1455, 1260 and 1185$cm^{-1}$, $\delta[(CD_3)_2CO]$ 1.23 [9H, t, J 7Hz, $\overset{\oplus}{H}N(CH_2\underline{CH_3})_3$] 1.35, 1.51 (6H, 2xs, $2x2CH_3$) 2.32, 2.37 (6H, 2xs, $2xCH_3CO$), 3.15 [6H, q, J 7Hz, $\overset{\oplus}{H}N(\underline{CH_2}CH_3)_3$], 3.55 (3H, s, $OCH_3$), 4.50 (1H, s, 3H), 5.22 (2H, s, $O\underline{CH_2}Ph$), 5.43 (1H, s, 5H), 5.62 (1H, s, CHCON), 7.37 (5H, m, Ph), 7.87 (1H, s, Ar), 9.30 (1H, s, CONH).

v) Disodium 6,β-[R-2-(4,5-diacetoxy-2,3-dichlorophenyl)-2-sulphoacetamido]-6,α-methoxypenicillanate

Prepared as described in example 4 v except that benzyl 6,β-[R-2-(4,5-diacetoxy-2,3-dichlorophenyl)-2-sulphoacetamido]-6,α-methoxypenicillanate (0.56g) was used and the mixture passed through a column of Dowex 50W-8X resin before being freeze dried to yield 0.29g, 59.2%,

$\nu_{max}$ (KBr) 3450 (br), 1765, 1685, 1610, 1455, 1370 and 1200 (br)$cm^{-1}$, $\delta(D_2O)$ 1.15, 1.27 (6H, 2xs, 2x $CH_3$), 2.29, 2.33 (6H, 2xs, 2x$CH_3CO$), 3.52 (3H, s, $OCH_3$), 4.18 (1H, s, 3H), 5.42, 5.75 (2H, 2xs, CHCON, 5H), 7.95 (1H, s, Ar).

## Biological Data

Antibacterial activities of a number of compounds described (MIC µg/ml).

| Organism | Compound of Example No. | | | | | | |
|---|---|---|---|---|---|---|---|
| | 4v | 4vi | 5 | 6vi | 6vii | 7 | 8 |
| E. coli ESS | 0.05 | 0.05 | 0.2 | 4.0 | 32 | 0.5 | 0.25 |
| E. coli JT4 | 0.25 | 0.5 | 0.5 | 16 | 64 | 8.0 | 1.0 |
| Ps. aeruginosa 10662 | 0.5 | 2.5 | 5.0 | 16 | 128 | 16 | 2.0 |
| Ps. aeruginosa Dalgleish | | | | 4.0 | 128 | 8.0 | 2.0 |
| Ps. aeruginosa $10^{-2}$ | 0.25 | 0.5 | 1.0 | | | | |
| N. catarrbalis 1502 | <0.01 | <0.02 | <0.02 | | | | |

0091302

A.

CLAIMS

1. A compound of formula (I) or a pharmaceutically acceptable salt or in vivo hydrolysable ester thereof:

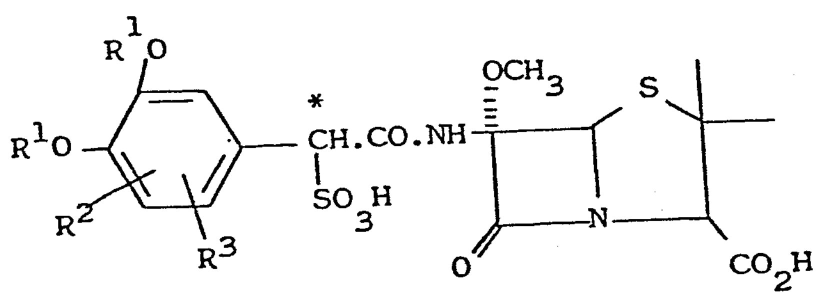

(I)

wherein $R^1$ is hydrogen or $C_{1-6}$ alkylcarbonyl or $C_{1-6}$ alkoxycarbonyl; $R_2$ is hydrogen, halogen, hydroxy, $C_{1-6}$ alkoxy, nitro, amino, $C_{1-6}$ alkyl, $C_{1-6}$ haloalkyl, $C_{1-6}$ alkylcarbonyloxy, $C_{1-6}$ alkyl sulphonylamino, carboxy, cyano, or $C_{1-6}$ alkoxycarbonyl; $R_3$ is halogen, hydroxy, $C_{1-6}$ alkoxy, nitro, amino, $C_{1-6}$ alkyl, $C_{1-6}$ haloalkyl, $C_{1-6}$ alkylcarbonyloxy, or $C_{1-6}$ alkyl sulphonyl-amino, carboxy, cyano, or $C_{1-6}$ alkyloxycarbonyl.

2. A compound as claimed in claim 1 wherein $R^2$ is hydrogen, halogen, hydroxy, $C_{1-6}$ alkoxy, nitro, $C_{1-6}$ alkylcarbonyloxy, carboxy or $C_{1-6}$ alkoxycarbonyl.

3. A compound as claimed in either of claims 1 or 2 wherein $R^3$ is halogen, hydroxy, $C_{1-6}$ alkoxy, nitro, amino, $C_{1-6}$ alkylcarbonyloxy, carboxy or $C_{1-6}$ alkyl.

4. A compound as claimed in any of claims 1 to 3 wherein $R^3$ is chlorine, bromine, hydroxy, amino, nitro, cyano, carboxy, methyl, ethyl or acetoxy.

5. A compound as claimed in any of claims 1 to 5 wherein $R^1$ is hydrogen or acetyl.

6. A compound selected from the following, or a pharmaceutically acceptable salt or in vivo hydrolysable ester thereof:

6,β-[2-(3,4-dihydroxy-5-nitrophenyl)-2-sulphoacetamido]-6,α-methoxypenicillanic acid; or

6,β-[2-(5-amino-3,4-dihydroxyphenyl)-2-sulphoacetamido]-6,α-methoxypenicillanic acid; or

6,β-[2-(3-chloro-4,5-dihydroxyphenyl)-2-sulphoacetamido]-6,α-methoxypenicillanic acid; or

6,β-[2-(2-chloro-4,5-diacetoxyphenyl)-2-sulphoacetamido-6,α-methoxypenicillanic acid; or

6,β-[2-(4,5-diacetoxy-2-methylphenyl)-2-sulphoacetamido]-6,α-methoxypenicillanic acid; or

6,β-[2-(4,5-diacetoxy-3-methylphenyl)-2-sulphoacetamido-6,α-methoxypenicillanic acid; or

6,β-[2-(4,5-diacetoxy-2,3-dichlorophenyl)-2-sulphoacetamido-6,α-methoxypenicillanic acid.

7. A process for the preparation of a compound as claimed in any of claims 1-6 which process comprises:

A) reacting a compound of formula (III):

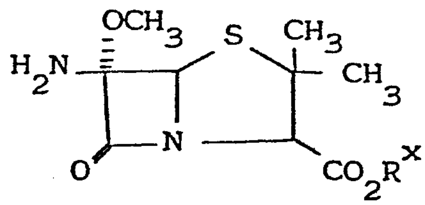

(III)

wherein the amino group is optionally substituted with a group which permits acylation to take place, and wherein $R^x$ is hydrogen or a carboxyl-blocking group, with an N-acylating derivative or an acid of formula (IV):

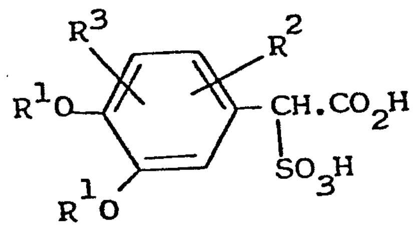

(IV)

wherein $R^1$, $R^2$ and $R_3$ are defined with respect to formula (I) above and wherein any reactive groups may be protected;

B) reacting a compound of formula (V):

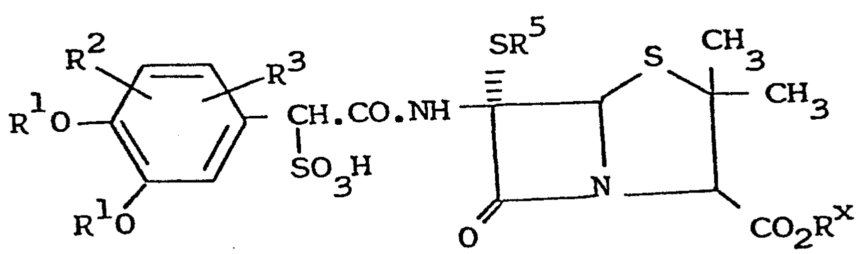

(V)

wherein $R^1$, $R^2$, $R^3$ and $R^x$ are as defined above and $R^5$ is $C_{1-6}$ alkyl, aryl or benzyl; with methanol in the presence of a metal ion;

C)

a) treating a compound of formula (VI):

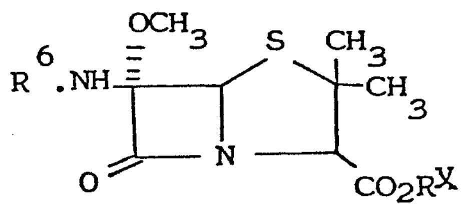

(VI)

wherein $R^x$ is a carboxyl-blocking group, and $R^6$ is an acyl group, in particular an acyl group derived from the side-chain of a natural penicillin, such as benzyl penicillin or phenoxymethyl penicillin; with an agent forming an imino halide;

b) treating the imino halide with a compound to introduce a group $QR_f$ on the imino carbon atom, wherein Q is oxygen, sulphur or nitrogen and $R_f$ is an alkyl group of from 5 to 14 carbon atoms, to form an iminoether, iminothioether, or amidine (when Q is O, S, or N resp ectively);

c) reacting with an N-acylating derivative of an acid of formula (IV) and wherein any reactive groups may be protected;

d) treating with water; and after step (A), (B), or (C), if necessary carrying out one or more of the following steps:

i) removing any carboxyl-blocking group $R^x$;

ii) removing any substituent on the amide group;

iii) removing any protecting group on the acid of formula (IV);

iv) converting the product into a salt or in vivo hydrolysable ester thereof.

8. A composition which comprises a compound as claimed in any one of claims 1 to 6 together with a pharmaceutically acceptable carrier or excipient.

9. A composition as claimed in claim 8 which also comprises a β-lactamase inhibitor.

10. A compound as claimed in any of claims 1-6 for use in the treatment of antibacteral infections in the human or animal body.

European Patent Office

# PARTIAL EUROPEAN SEARCH REPORT

which under Rule 45 of the European Patent Convention shall be considered, for the purposes of subsequent proceedings, as the European search report

0091302

Application number

EP 83 30 1876

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| Y | EP - A - 0 015 690 (BEECHAM)<br>* pages 31-32; example 11; claims 1,2,4,7,9,10 *<br>-- | 1,5,7,8 | C 07 D 499/00<br>499/62<br>A 61 K 31/43//<br>C 07 C 79/38<br>79/24<br>59/00 |
| P,Y | EP - A - 0 066 373 (BEECHAM)<br>* claims *<br>------- | 1,5,7-9 | |

TECHNICAL FIELDS SEARCHED (Int. Cl. 3)

C 07 D 499/00
A 61 K 31/00

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 1-9

Claims searched incompletely:

Claims not searched: 10

Reason for the limitation of the search:

Method for treatment of the human or animal body by surgery or therapy (see article 52(4) of the European Patent Convention).

| Place of search | Date of completion of the search | Examiner |
|---|---|---|
| The Hague | 01-07-1983 | CHOULY |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

EPO Form 1505.1. 03.82